(19) Europäisches Patentamt
European Patent Office
Office européen des brevets

(11) EP 0 821 949 B1

(12) EUROPÄISCHE PATENTSCHRIFT

(45) Veröffentlichungstag und Bekanntmachung des Hinweises auf die Patenterteilung:
**30.06.2004 Patentblatt 2004/27**

(51) Int Cl.[7]: **A61K 7/50**, A61K 7/00

(21) Anmeldenummer: **97112203.1**

(22) Anmeldetag: **17.07.1997**

(54) **Schaumförmige Zubereitungen mit einem Gehalt an grenzflächenaktiven Substanzen und Elektrolyten**

Foam compositions containing surface active agents and electrolytes

Composition sous forme de mousse contenant des tensioactifs et des électrolytes

(84) Benannte Vertragsstaaten:
**AT BE CH DE ES FR GB IT LI NL SE**

(30) Priorität: **02.08.1996 DE 19631220**

(43) Veröffentlichungstag der Anmeldung:
**04.02.1998 Patentblatt 1998/06**

(73) Patentinhaber: **Beiersdorf Aktiengesellschaft 20245 Hamburg (DE)**

(72) Erfinder:
- **Gers-Barlag, Heinrich, Dr. 25495 Kummerfeld (DE)**
- **Müller, Anja 20253 Hamburg (DE)**
- **Maurer, Peter 22880 Wedel (DE)**

(56) Entgegenhaltungen:
**WO-A-92/01508**
**WO-A-96/22073**
**DE-A- 19 547 679**
**US-A- 4 683 242**
**US-A- 5 306 486**
**US-A- 5 322 683**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 821 949 B1

## Beschreibung

Die vorliegende Erfindung betrifft schaumförmige oder schäumbare kosmetische und dermatologische Zubereitungen, insbesondere hautpflegende kosmetische und dermatologische Zubereitungen. In einer besonderen Ausführungsform betrifft die vorliegende Erfindung auch nachschäumende kosmetische und dermatologische Zubereitungen.

Schaumförmige oder schäumbare kosmetische Zubereitungen sind an sich bekannt. Schäume erlauben eine feine Verteilung von Wirkstoffen auf der Haut. Allerdings sind Schäume in der Regel nur durch Verwendung besonderer Tenside, welche darüberhinaus oft wenig hautverträglich sind, zu erreichen.

In einer besonderen Ausführungsform die vorliegende Erfindung betrifft die vorliegende Erfindung kosmetische Reinigungsmittel.

Derartige Mittel sind an sich bekannt. Es handelt sich dabei im wesentlichen um oberflächenaktive Substanzen oder Stoffgemische, die dem Verbraucher in verschiedenen Zubereitungen angeboten werden.

Zubereitungen dieser Art sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder und dergleichen.

Oberflächenaktive Stoffe - am bekanntesten die Alkalisalze der höheren Fettsäuren, also die klassischen "Seifen" - sind amphiphile Stoffe, die organische unpolare Substanzen in Wasser emulgieren können.

Diese Stoffe schwemmen nicht nur Schmutz von Haut und Haaren, sie reizen, je nach Wahl des Tensids oder des Tensidgemisches, Haut und Schleimhäute mehr oder minder stark.

Das gebräuchlichste Tensid für kosmetische Zusammensetzungen ist das Natriumlaurylethersulfat. Obwohl es gute Waschkraft besitzt und einigermaßen hautund schleimhautverträglich ist, sollten empfindliche Personen den häufigen Kontakt damit meiden.

Es ist zwar eine große Zahl recht milder Tenside erhältlich, jedoch sind die Tenside des Standes der Technik entweder mild, reinigen aber schlecht, oder aber sie reinigen gut, reizen jedoch Haut oder Schleimhäute.

Nachschäumende Zubereitungen liegen oft in speziellen Ausführungsformen wie etwa nachschäumenden Rasiergelen oder dergleichen vor. Solche werden zunächst in fließförmiger Form aus einem Aerosolbehälter auf die Haut aufgetragen und entwickeln erst dort nach kurzer Verzögerung unter dem Einfluß des enthaltenen Nachschäummittels den eigentlichen Schaum, beispielsweise einen Rasierschaum. Der Vorteil solcher Zubereitungen gegenüber fertigen Schäumen beispielsweise, welche bereits geschäumt aus dem Aerosolbehälter auf die Haut aufgebracht werden, liegt in einer besseren Benetzung. Bei einem Rasierschaum ist dies vorteilhaft, da vollständigere Befeuchtung des Bartes bewirkt, daß der Rasierapparat besser gleitet.

Nachschäumende Zubereitungen sind also an sich bekannt. Die US-PS 3,541,581 nennt als wesentliche Bestandteile einer solchen Zubereitung Wasser, Seife, (also wasserlösliche Salze höherer Fettsäuren), Gelstrukturbildner und Nachschäummittel. Auch andere derartige Zubereitungen sind bekannt, die aber alle den Nachteil haben, kosmetisch unelegant zu sein und/oder die Anforderung an niedriges Reizpotential nicht zu erfüllen.

US-5,322,683 offenbart schäumbare Aerosol zusammen selzungen, welche Methylglucosesesquistearat, acetylierte, Monoglycerid und Bentonit enthalten, jedoch wasserfrei sind.

Aufgabe der vorliegenden Aufgabe war also, den Stand der Technik zu bereichern und seinen Nachteilen abzuhelfen.

Es war indes überraschend und für den Fachmann nicht vorauszusehen, daß Wirkstoffkombinationen aus

(a) einer oder mehreren grenzflächenaktiven Substanzen, gewählt aus der Gruppe der Glucosederivate, welche sich durch die Strukturformel

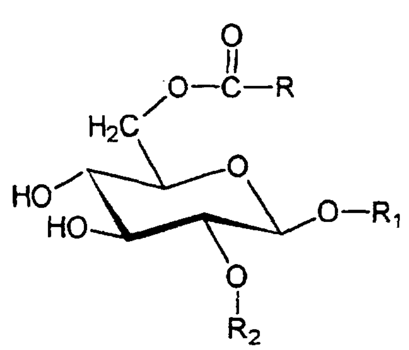

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

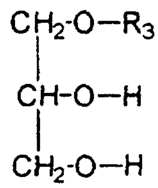

bzw.

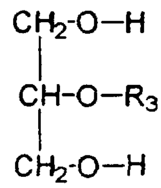

wiedergegeben, wobei $R_3$ einen verzweigten oder unverzweigten Acylrest mit 6 - 24 Kohlenstoffatomen darstellt,

(c) einem oder mehreren kosmetisch oder pharmazeutisch akzeptablen Elektrolyten

den Nachteilen des Standes der Technik abhelfen.

Insbesondere wird die vorliegende Erfindung verkörpert durch kosmetische oder pharmazeutische Zubereitungen, welche Wirkstoffkombinationen aus

(a) einer oder mehreren grenzflächenaktiven Substanzen, gewählt aus der Gruppe der Glucosederivate, welche sich durch die Strukturformel

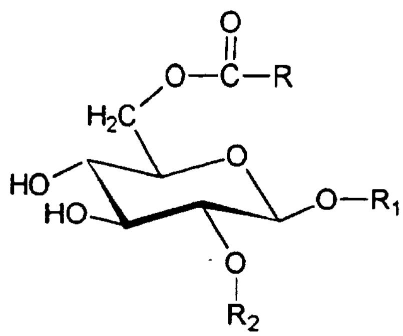

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

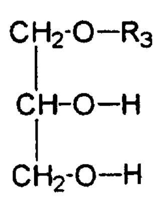

bzw.

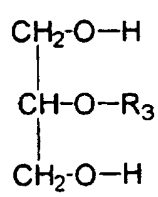

wiedergegeben, wobei $R_3$ einen verzweigten oder unverzweigten Acylrest mit 6 - 24 Kohlenstoffatomen darstellt,

(c) einem oder mehreren kosmetisch oder pharmazeutisch akzeptablen Elektrolyten,

(d) einer Wasserphase, welche gegebenenfalls übliche, darin lösliche und/oder dispergierbare Substanzen, darunter insbesondere weitere kosmetisch oder pharmazeutisch akzeptable Substanzen, enthält, und

(e) gegebenenfalls einer Ölphase, welche gegebenenfalls übliche, darin lösliche und/oder dispergierbare kosmetisch oder pharmazeutisch akzeptable Substanzen enthält,

enthalten und in Form von Schäumen vorliegen.

Insbesondere stellt die Verwendung von Wirkstoffkombinationen aus

(a) einer oder mehreren grenzflächenaktiven Substanzen, gewählt aus der Gruppe der Glucosederivate, welche sich durch die Strukturformel

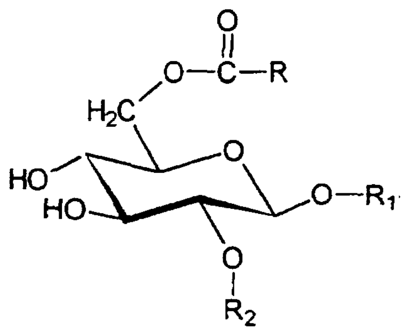

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

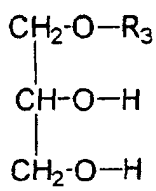

bzw.

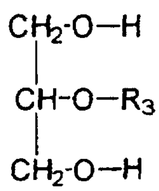

wiedergegeben, wobei $R_3$ einen verzweigten oder unverzweigten Acylrest mit 6 - 24 Kohlenstoffatomen darstellt.

(c) einem oder mehreren kosmetisch oder pharmazeutisch akzeptablen Elektrolyten.

(d) einer Wasserphase, welche gegebenenfalls übliche, darin lösliche und/oder dispergierbare Substanzen, darunter insbesondere weitere kosmetisch oder pharmazeutisch akzeptable Substanzen, enthält, und

(e) gegebenenfalls einer Ölphase, welche gegebenenfalls übliche, darin lösliche und/oder dispergierbare kosmetisch oder pharmazeutisch akzeptable Substanzen enthält,

zur Erzeugung kosmetischer oder pharmazeutischer Zubereitungen in Schaumform eine vorteilhafte Verkörperung der vorliegenden Erfindung dar.

Vorteilhaft wird R gewählt aus der Gruppe der unverzweigten Alkylreste, wobei der Myristylrest, der Palmitylrest, der Stearylrest und der Eicosylrest bevorzugt werden.

$R_1$ kann vorteilhaft ein Wasserstoffatom darstellen, wird aber bevorzugt aus der Gruppe Methyl-, Ethyl-, Propyl- und Isopropyl- gewählt.

$R_2$ kann vorteilhaft ein Wasserstoffatom darstellen, kann aber gleichermaßen vorteilhaft aus der Gruppe Myristoyl-, Palmitoyl-, Stearoyl- und Eicosoyl- gewählt werden.

Besonders vorteilhaft wird als grenzflächenaktive Substanzen aus der Gruppe der Glucosederivate das Methylglucosesesquistearat gewählt, welches zu etwa gleichen Teilen aus den Substanzen

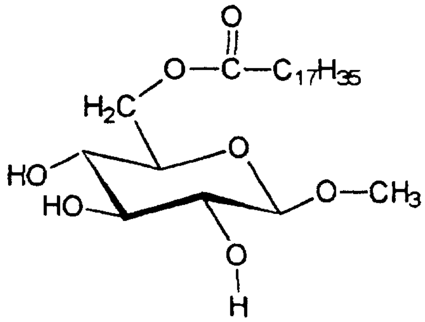

und

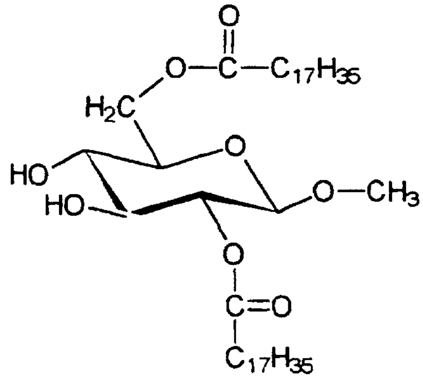

besteht. Solche Mischungen sind im Handel beispielsweise unter der Warenbezeichnung Tego® Care PS von der Gesellschaft Th.Goldschmidt KG erhältlich.

$R_3$ stellt bevorzugt einen Myristylrest, Palmitylrest, Stearylrest oder Eicosylrest dar.

Insbesondere vorteilhaft im Sinne der vorliegenden Erfindung hat sich als unter Punkt (b) aufgeführter grenzflächenaktiver Substanzen das Glycerylstearat herausgestellt, welches im Handel beispielsweise unter der Warenbezeichnung Tegin® M von der Gesellschaft Th.Goldschmidt KG erhältlich ist.

Die erfindungsgemäßen Zubereitungen enthalten Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Es ist auch gegebenenfalls vorteilhaft, Aluminiumchlorhydrat zu verwenden. Produkte sieser Art eignen sich dann besonders vorteilhaft als Körperdesodorantien.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Erfindungsgemäße Zusammensetzungen stellen ungeschäumt, also unmittelbar nach Zusammengeben der Einzelkomponenten, Zweiphasensysteme, in der Regel Emulsionen, dar. Sie können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.

Es hat sich darüberhinaus in überraschender Weise herausgestellt, daß bei der Verwendung von Treibmitteln, besonders vorteilhaft von in der gegebenenfalls vorhandenen Ölphase löslichen Treibmitteln, also beispielsweise üblichen Propan-Butan-Gemischen, die erfindungsgemäßen Zubereitungen nicht einfach als Aerosoltröpfchen versprüht werden, sondern sich zu feinblasigen, reichhaltigen Schäumen entwickeln, sobald solche mit solchen Treibmitteln beladenen Systeme Druckentspannung erfahren.

Solche nachschäumenden Zubereitungen werden daher ebenfalls als vorteilhafte Verkörperungen der vorliegenden Erfindung mit eigenständiger erfinderischer Tätigkeit angesehen.

Erfindungsgemäß sind feinblasige, reiche Schäume von hervorragender kosmetischer Eleganz erhältlich. Weiterhin sind erfindungsgemäß besonders gut hautverträgliche Zubereitungen erhältlich, wobei wertvolle Inhaltsstoffe besonders gut auf der Haut verteilt werden können.

Die erfindungsgemäßen kosmetischen und/oder dermatologischen Zubereitungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Eine weitere vorteilhafte Ausführungsform der vorliegenden Erfindung besteht in After-Sun-Produkten, insbesondere als Pflegeschäume mit kühlender Wirkung.

Erfindungsgemäß können schaumförmige oder schäumbare kosmetische und dermatologische Zubereitungen auch als Reinigungsmittel eingesetzt werden.

Auch als Rasiermittel, beispielsweise Rasierschäume, aber auch sonstigen Preund Aftershave-Zubereitungen findet die Erfindung erfolgreich Anwendung.

Kosmetische Zubereitungen, die ein Hautreinigungsmittel oder Shampoonierungsmittel darstellen, enthalten vorzugsweise zusätzlich mindestens eine anionische, nicht-ionische oder amphotere oberflächenaktive Substanz, oder auch Gemische aus solchen Substanzen, erfindungsgemäße Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 94 Gew.-%, insbesondere bis zu 50 Gew.-% in dem Shampoonierungsmittel vorliegen.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält gegebenenfalls zusätzlich mindestens ein kationisches, anionisches, nichtionisches oder amphoteres Polymer oder auch Gemische derselben, sowie erfindungsgemäße Wirkstoffkombinationen in wirksamer Konzentration. Die Menge der verwendeten Polymeren liegt z.B. zwischen 0,1 und 10 Gew.-%, bevorzugt zwischen 0,1 und 3 Gew.-%.

Erfindungsgemäß können kosmetische Zubereitungen zur Behandlung und Pflege der Haut und Haare neben einem wirksamen Gehalt an erfindungsgemäßen Wirkstoffkombinationen und dafür üblicherweise verwendeten Lösungsmitteln, bevorzugt Wasser, noch Gelbildner, z.B. organische Verdickungsmittel, z.B. Gummiarabikum, Xanthangummi, Natriumalginat, Cellulose-Derivate, vorzugsweise Methylcellulose. Hydroxymethylcellulose, Hydroxyethylcellulose, Hydroxypropylcellulose, Hydroxypropylmethylcellulose oder anorganische Verdckkungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in der Zubereitung z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Wirkstoffkombinationen in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Kosmetische Zubereitungen, die kosmetische Reinigungszubereitungen für die Haut darstellen, können in flüssiger oder fester Form vorliegen. Sie enthalten neben erfindungsgemäßen Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.

Besonders vorteilhaft ist ein Zusatz vonölllöslichen UV-Filtern und/oder UV-Strahlung absorbierender bzw. reflektierender anorganischer Pigmente.

Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte öllösliche UVA-Filtersubstanzen sind z.B.:

- Derivate des Dibenzoylmethans, vorzugsweise 1-(4'-tert.Butylphenyl)-3-(4'-methoxyphenyl)propan-1,3-dion
- 1-Phenyl-3-(4'-isopropylphenyl)propan-1,3-dion.

Erfindungsgemäße kosmetische und dermatologische Zubereitungen enthalten außerdem vorteilhaft, aber nicht zwingend, anorganische Pigmente auf Basis von Metalloxiden und/oder anderen in Wasser schwerlöslichen oder

unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Die anorganischen Pigmente liegen bevorzugt in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \text{ -> } n\ TiO_2\ \text{(oberfl.)}$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa oder MT 100 T von der Firma Tayca oder M 160 von der Frima Kemira erhältlich.

Als wasserdispergierbare anorganische Mikropigmente könnnen beispielsweise solche Produkte gewählt werden, welche unter der Handelsbezeichnung Tioveil® von der Firma Tioxide erhältlich sind.

Die Gesamtmenge an anorganischen Pigmenten, insbesondere hydrophoben anorganischen Mikropigmenten (als an sich fakultativ einzusetzender zusätzlicher Substanz) in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0.05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Ölphase der erfindungsgemäßen Zubereitungen wird vorteilhaft gewählt aus der Gruppe der Ester aus

gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| Beispiel 1 | |
|---|---|
| | Gew.-% |
| Glycerylstearat | 2,00 |
| Methylglucosesesquistearat | 5,00 |
| Caprylic/Capric Triglyceride | 5,00 |
| Octyldodecanol | 5,00 |
| Dicaprylylether | 2.00 |
| Glycerin | 3,00 |
| Tocopherylacetat | 1.00 |

(fortgesetzt)

| Beispiel 1 | |
|---|---|
| | Gew.-% |
| $MgSO_4$ | 1,00 |
| Parfum, Konservierungsmittel | q.s. |
| Wasser | ad 100,00 |

| Beispiel 2 | |
|---|---|
| | Gew.-% |
| Glycerylstearat | 2,00 |
| Methylglucosesesquistearat | 5,00 |
| Dicaprylylether | 5,00 |
| Glycerin | 3,00 |
| 4-Methylbenzylidencampher | 0,50 |
| NaCl | 2,00 |
| Parfum, Konservierungsmittel | q.s. |
| Wasser | ad 100,00 |

| Beispiel 3 | |
|---|---|
| | Gew.-% |
| Glycerylstearat | 2,00 |
| Methylglucosesesquistearat | 5,00 |
| Caprylic/Capric Triglyceride | 1,67 |
| Octyldodecanol | 1,67 |
| Dicaprylylether | 1,67 |
| Butylenglycol | 3,00 |
| 4-Methylbenzylidencampher | 1,00 |
| Tocopherylacetat | 1,00 |
| $ZnSO_4$ | 2,00 |
| NaCl | 2,00 |
| Parfum, Konservierungsmittel | q.s. |
| Wasser | ad 100,00 |

| Beispiel 4 | |
|---|---|
| | Gew.-% |
| Treibmittel (Isobutan) | 5,00 |
| Glycerylstearat | 2,00 |
| Methylglucosesesquistearat | 5,00 |
| Caprylic/Capric Triglyceride | 1,67 |
| Octyldodecanol | 1,67 |
| Dicaprylylether | 1,67 |
| Butylenglycol | 3,00 |
| 4-Methylbenzylidencampher | 1,00 |
| Tocopherylacetat | 1,00 |
| $ZnSO_4$ | 2,00 |
| NaCl | 2,00 |

(fortgesetzt)

| Beispiel 4 | |
|---|---|
| | Gew.-% |
| Parfum, Konservierungsmittel | q.s. |
| Wasser | ad 100,00 |

| Beispiel 5 | |
|---|---|
| | Gew.-% |
| Glycerylstearat | 2,00 |
| Methylglucosesesquistearat | 5,00 |
| Caprylic/Capric Triglyceride | 5,00 |
| Octyldodecanol | 5,00 |
| Dicaprylylether | 1,67 |
| Jojobaöl | 3,00 |
| Glycerin | 3,00 |
| Tocopherylacetat | 1,00 |
| $ZnSO_4$ | 2,00 |
| $MgSO_4$ | 2,00 |
| ZnO | 1,00 |
| Parfum, Konservierungsmittel | q.s. |
| Wasser | ad 100,00 |

| Beispiel 6 | |
|---|---|
| | Gew.-% |
| Glycerylstearat | 2,00 |
| Methylglucosesesquistearat | 5,00 |
| Caprylic/Capric Triglyceride | 1,67 |
| Octyldodecanol | 1,67 |
| Dicaprylylether | 1,67 |
| Butylenglycol | 5,00 |
| Tocopherylacetat | 1,00 |
| NaCl | 2,00 |
| NaOH | q.s. |
| Parfum, Konservierungsmittel | q.s. |
| Wasser | ad 100,00 |

## Patentansprüche

**1.** Verwendung von Zubereitungen, **gekennzeichnet dadurch, daß** sie Wirkstoffkombinationen aus

(a) einer oder mehreren grenzflächenaktiven Substanzen, gewählt aus der Gruppe der Glucosederivate, welche sich durch die Strukturformel

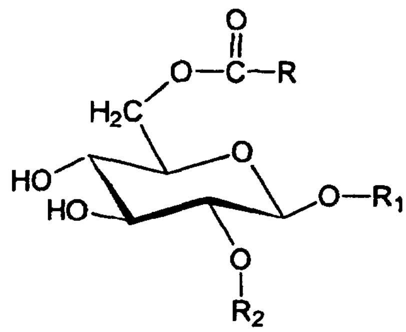

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

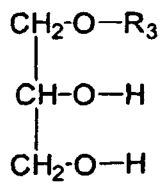

bzw.

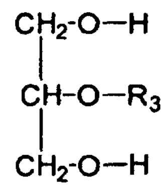

wiedergegeben, wobei $R_3$ einen verzweigten oder unverzweigten Acylrest mit 6 - 24 Kohlenstoffatomen darstellt,

(c) einem oder mehreren kosmetisch oder pharmazeutisch akzeptablen Elektrolyten, welcher oder welche gewählt werden aus der Gruppe der Salze mit folgenden Anionen: Chloride, ferner anorganische Oxo-Element-Anionen sowie der auf organischen Anionen basierende Elektrolyte,

enthalten, und ferner aus

(d) einer Wasserphase, welche gegebenenfalls übliche, darin lösliche und/oder dispergierbare Substanzen, darunter insbesondere weitere kosmetisch oder pharmazeutisch akzeptable Substanzen, enthält, und

(e) einer Ölphase, welche gegebenenfalls übliche, darin lösliche und/oder dispergierbare kosmetisch oder pharmazeutisch akzeptable Substanzen enthält,

zur Erzeugung von Schäumen.

2. Verwendung nach Anspruch 1, **dadurch gekennzeichnet, daß** in der Strukturformel des oder der grenzflächenaktiven Substanzen aus der Gruppe der Glucosederivate der Rest R gewählt wird aus der Gruppe Myristyl-, Palmityl-, Stearyl-, Eicosyl-, der Rest $R_1$ ein Wasserstoffatom darstellt oder aus der Gruppe Methyl-, Ethyl-, Propylund Isopropyl- gewählt wird, und/oder der Rest $R_2$ ein Wasserstoffatom darstellt oder aus der Gruppe Myristoyl-, Palmitoyl-, Stearoyl-, Eicosoyl- gewählt wird.

3. Verwendung nach Anspruch 1, **dadurch gekennzeichnet, daß** als grenzflächenaktive Substanz aus der Gruppe der Glucosederivate das Methylglucosesesquistearat gewählt wird.

4. Verwendung nach Anspruch 1, **dadurch gekennzeichnet, daß** als grenzflächenaktive Substanz aus der Gruppe der Glycerin-mono- bzw. -dicarbonsäure-monoester das Glycerylstearat gewählt wird.

## Claims

1. Use of preparations, **characterized in that** they contain active substance combinations of

(a) one or more surface-active substances selected from the group consisting of glucose derivatives of the structural formula

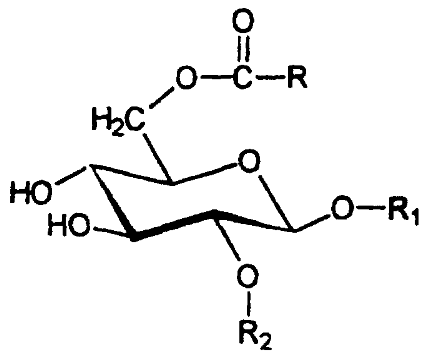

in which R is a branched or unbranched alkyl radical having 1 to 24 carbon atoms, $R_1$ is either a hydrogen atom or a branched or unbranched alkyl radical having 1 to 24 carbon atoms and $R_2$ is either a hydrogen atom or a branched or unbranched acyl radical having 1 to 24 carbon atoms,

(b) one or more surface-active substances selected from the group consisting of glycerol monocarboxylic or dicarboxylic acid monoesters of the general formula

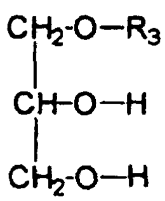

or

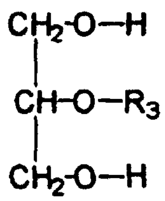

in which $R_3$ is a branched or unbranched acyl radical having 6 - 24 carbon atoms, and

(c) one or more cosmetically or pharmaceutically acceptable electrolytes, which is/are selected from the group of salts having the following anions: chlorides, also inorganic oxo anions and of electrolytes based on organic anions,

and furthermore of

(d) an aqueous phase which may contain conventional substances soluble and/or dispersible therein, including especially other cosmetically or pharmaceutically acceptable substances, and

(e) an oil phase which may contain conventional cosmetically or pharmaceutically acceptable substances soluble and/or dispersible therein,

for the production of foams.

**2.** Use according to Claim 1, **characterized in that**, in the structural formula of the surface-active substance or substances from the group consisting of glucose derivatives, the radical R is selected from the group consisting of myristyl, palmityl, stearyl and eicosyl, the radical $R_1$ is a hydrogen atom or is selected from the group consisting of methyl, ethyl, propyl and isopropyl, and/or the radical $R_2$ is a hydrogen atom or is selected from the group consisting of myristoyl, palmitoyl, stearoyl and eicosoyl.

**3.** Use according to Claim 1, **characterized in that** methyl glucose sesquistearate is selected from the group consisting of glucose derivatives as surface-active substance.

**4.** Use according to Claim 1, **characterized in that** glyceryl stearate is selected as the surface-active substance from the group consisting of glycerol monocarboxylic or dicarboxylic acid monoesters.

## Revendications

1. Utilisation de compositions, **caractérisée en ce qu'**elles contiennent des combinaisons de substances actives constituées par

   a) une ou plusieurs substances tensioactives, choisies dans le groupe des dérivés du glucose, qui sont **caractérisés par** la formule de structure

   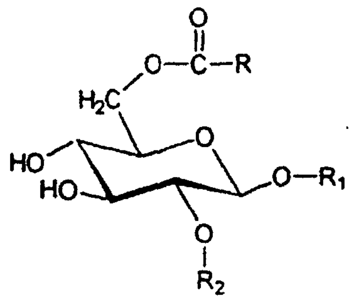

   R représentant un radical alkyle ramifié ou non ramifié, comprenant 1 à 24 atomes de carbone, $R_1$ représentant un atome d'hydrogène ou un radical alkyle ramifié ou non ramifié comprenant 1 à 24 atomes de carbone et $R_2$ représentant un atome d'hydrogène ou un radical acyle ramifié ou non ramifié comprenant 1 à 24 atomes de carbone,
   (b) une ou plusieurs substances tensioactives, choisies dans le groupe des monoesters d'acide monocarboxylique ou dicarboxylique de glycérol de formule générale

   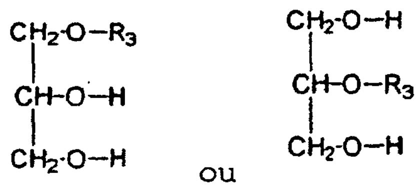

   $R_3$ représentant un radical acyle ramifié ou non ramifié comprenant 6 à 24 atomes de carbone,
   (c) un ou plusieurs électrolytes cosmétiquement ou pharmaceutiquement acceptables, choisi ou choisis dans le groupe constitué par les sels avec les anions suivants : les chlorures, en outre les anions d'oxo-éléments inorganiques ainsi que les électrolytes à base d'anions organiques
   en outre par
   (d) une phase aqueuse, qui contient le cas échéant des substances usuelles qui y sont solubles et/ou dispersibles, parmi celles-ci en particulier d'autres substances cosmétiquement ou pharmaceutiquement acceptables et
   (e) le cas échéant une phase huileuse, qui contient le cas échéant des substances cosmétiquement ou pharmaceutiquement acceptables qui y sont solubles et/ou dispersibles,

   pour la production de mousses.

2. Utilisation selon la revendication 1, **caractérisée en ce que** dans la formule de structure de la ou des substances tensioactives du groupe des dérivés de glucose, le radical R est choisi dans le groupe constitué par les radicaux myristyle, palmityle, stéaryle, eicosyle, le radical $R_1$ représente un atome d'hydrogène ou est choisi dans le groupe constitué par les radicaux méthyle, éthyle, propyle et isopropyle et/ou le radical $R_2$ représente un atome d'hydrogène ou est choisi dans le groupe constitué par les radicaux myristoyle, palmitoyle, stéaroyle, eicosoyle.

3. Utilisation selon la revendication 1, **caractérisée en ce qu'**on choisit comme substance tensioactive du groupe des dérivés de glucose le glucosesesquistéarate de méthyle.

4. Utilisation selon la revendication 1, **caractérisée en ce qu'**on choisit comme substance tensioactive du groupe des monoesters de l'acide monocarboxylique ou dicarboxylique de glycérol le stéarate de glycéryle.